(19) Europäisches Patentamt
European Patent Office
Office européen des brevets

(11) Publication number: **0 109 195**
**B1**

(12) **EUROPEAN PATENT SPECIFICATION**

(45) Date of publication of patent specification: **11.01.89**

(51) Int. Cl.⁴: **C 07 F 7/08,** A 61 K 31/695

(21) Application number: **83306255.7**

(22) Date of filing: **14.10.83**

# (54) Antitumor organosilicon compounds and their preparation.

(30) Priority: **16.10.82 JP 181782/82**

(43) Date of publication of application:
**23.05.84 Bulletin 84/21**

(45) Publication of the grant of the patent:
**11.01.89 Bulletin 89/02**

(84) Designated Contracting States:
**CH DE FR GB IT LI**

(56) References cited:
**EP-A-0 046 242**

**CHEMICAL ABSTRACTS, vol. 71, no. 25, 22nd December 1969, page 481, no. 124572y, Columbus, Ohio, US; E. LUKEVICS et al.:"Nitrogen-containing organosilicon compounds. XXI. Organosilicon derivatives of 2 (diethylamine)ethanethiol" & ZH. OBSHCH. KHIM. 1969, 39(8), 1782-3**

**The file contains technical information submitted after the application was filed and not included in this specification**

(73) Proprietor: **SANWA KAGAKU KENKYUSHO CO., LTD.**
**No. 35, Higashi-sotobori-cho**
**Higashi-ku Nagoya-shi Aichi-ken (JP)**
(73) Proprietor: **Shin-Etsu Chemical Co., Ltd.**
**6-1, Ohtemachi 2-chome**
**Chiyoda-ku Tokyo 100 (JP)**

(72) Inventor: **Toyoshima, Shigeshi**
**14-22, Fiunabashi 3-chome**
**Setagaya-ku Tokyo (JP)**
Inventor: **Kurono, Masayasu**
**72-2, Kakeage-cho 2-chome**
**Minami-ku Nagoya-shi Aichi-ken (JP)**
Inventor: **Unno, Ryoichi**
**1-3, Hikarigaoka**
**Chigusa-ku Nagoya-shi Aichi-ken (JP)**
Inventor: **Ito, Koichi**
**3-32, Minami-cho 4-chome**
**Higashi Kurume-shi Tokyo (JP)**

(74) Representative: **Diamond, Bryan Clive et al**
**Gee & Co. Chancery House Chancery Lane**
**London WC2A 1QU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
SOVIET INVENTIONS ILLUSTRATED, Section I, Chemical, vol. V, no. 12, 1974, Derwent Publications, London, GB; & SU - A - 386 948 (IRKUTSK ORG. CHEM. INST. SIBER. BR. ACAD. SCIE. USSR. (22-10-1973)

SOVIET INVENTIONS ILLUSTRATED, Section I, Chemical, vol. V, no. 21, 1974, Derwent Publications, London, GB; & SU - A - 391 144 (ORG.CHEM. INST. SIBERIAN BR. ACAD. SCIE. USSR. 26-12-1973

**Description**

The present invention relates to novel organosilicon compounds and non-toxic salts thereof having an antitumor action and useful as pharmaceutical agents, a process for the preparation thereof as well as an antitumor agent comprising as an effective component at least one of the compounds and salts.

As silicon-containing compounds having the antitumor action, silatranes have hitherto been known but have a problem in use due to a relatively high toxicity thereof. Therefore, it has been desired to develop a new antitumor agent which shows a good therapeutic effect and has no or reduced toxicity.

Therefore, an object of the invention is to provide novel organosilicon compounds ans non-toxic salts thereof, which show a good antitumor action and a low toxicity.

Another object of the invention is to provide a process for the preparation of such compounds and salts.

A still other object of the invention is to provide a novel antitumor agent comprising as an effective component at least one of such compounds and salts.

The compounds according to the invention are of the general formula

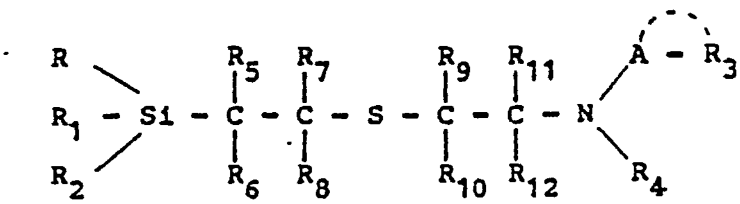

(I)

wherein A represents a carbonyl or sulfonyl group; R, $R_1$ and $R_2$ each represents an alkyl group; $R_3$ represents an alkyl, trifluoroalkyl, alkenyl, phenyl, p-chlorophenyl, p-methylphenyl, naphthyl, pyridyl or furyl or thienyl group;

$R_4$ represents a hydrogen atom, an alkyl or alkenyl group;

$R_5$, $R_6$, $R_7$, $R_8$, $R_9$, $R_{10}$, $R_{11}$ and $R_{12}$ each represents a hydrogen atom or an alkyl group;

or a non-toxic salt thereof.

EP—0046242 was filed by one of the present co-applicants, and it describes organosilicon compounds intended to have high anti-tumor activity and of low toxicity; these are of the general formula (I) given above wherein each of $R_1$ and $R_2$ is hydrogen or an optionally substituted hydrocarbon, and $R_5$ to $R_{12}$ are each hydrogen, but the substituents on the amino group are hydrogen or C1—3 alkyl. In particular, 2,(2-aminoethylthio) ethyl trimethyl silane, i.e. $(CH_3)_3—Si—(CH_2)_2—S—(CH_2)_2—NHCH_3$, is said to be a promising anti-tumor compound. However although the prior specification gives no toxicity data, we have now found that this compound has an acute toxicity higher than that of the corresponding maleate.

The present invention relates to the analogs of these known compounds wherein the amino group has an inserted carbonyl or sulfonyl group (A) and surprisingly these have been found to have much lower toxicity than said prior compound.

The invention includes the non-toxic salts thereof, such as the maleate.

Preferred classes of these compounds are those wherein R, $R_1$ and $R_2$ are each methyl and $R_5$ to $R_{12}$ are each hydrogen and A is carbonyl or sulfonyl or $—R_3$ is alkenyl and $R_4$ is hydrogen.

An alkyl group may be a linear alkyl radical having 1 to 7 carbon atoms, such as methyl, ethyl, *n*-propyl, *n*-butyl, *n*-pentyl, *n*-hexyl or *n*-heptyl or a branched alkyl radical, such as isopropyl, isobutyl, *sec*-butyl, *tert*-butyl or isopentyl. A substituted alkyl group may be a halogen-substituted alkyl radical, such as trifluoromethyl. An alkenyl group may be vinyl, allyl or isopropenyl. Suitable substituents for substituted phenyl are *o*-chloro, *p*-chloro, *p*-bromo, *p*-methyl and *p*-methoxy. Naphthyl and substituted naphthyl groups include 1-naphthyl, 2-naphthyl, 2-hydroxy-1-naphthyl and 2-methoxy-1-naphthyl. Pyridyl and substituted pyridyl include 2-pyridyl, 3-pyridyl, 4-pyridyl, 5-methyl-2-pyridyl and the like radicals. The furyl and substituted furyl may be 2-furyl, 3-furyl or 5-methyl-2-furyl. The thienyl and substituted thienyl may be 2-thienyl, 3-thienyl or 5-methyl-2-thienyl. A cycloalkyl group has 3 or more carbon atoms and may be cyclopropyl, cyclobutyl, cyclopentyl, cyclohexyl or cycloheptyl.

The compounds as shown by formula (I) can be prepared from the following compound (II) as the starting material.

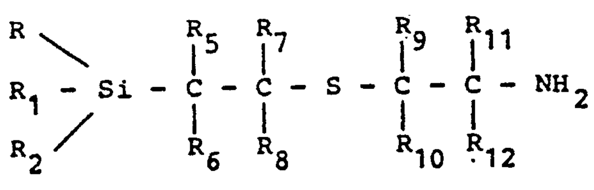

(II)

wherein R, $R_1$, $R_2$, $R_5$, $R_6$, $R_7$, $R_8$, $R_9$, $R_{10}$, $R_{11}$ and $R_{12}$ have the meanings defined above.

This starting material can easily be synthesized by subjecting a compound represented by the formula

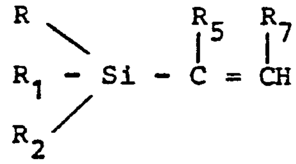

(III)

wherein R, $R_1$, $R_2$, $R_5$ and $R_7$ have the meanings defined above, to reaction with a compound represented by the formula

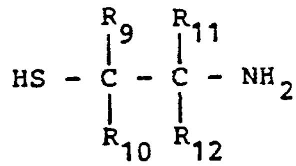

(IV)

wherein $R_9$, $R_{10}$, $R_{11}$ and $R_{12}$ have the meaning defined above,
in a polar organic solvent under ultraviolet irradiation and in the presence of a photochemical sensitizer.

(1) The compounds as shown by formula (I), wherein A is carbonyl group, can be prepared by subjecting the compound as shown by formula (II) to a reaction with an acid halide represented by a formula

$$R_3\text{—}COX \qquad (V)$$

or with an acid anhydride represented by a formula

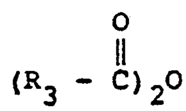

(VI)

wherein $R_3$ has the meaning defined above and X is halogen,
in equi-molar amount. It is, in general, preferable to carry out the reaction in a solvent, in the presence of a base and at a temperature of from 0°C to the boiling point of the used solvent. As the solvent, methylene chloride, chloroform, carbon tetrachloride or the like halogenohydrocarbon; ethyl ether, tetrahydrofuran, 1,4-dioxane or the like ether; or benzene, toluene or the like aromatic hydrocarbon may be used. As the base, triethylamine, pyridine, sodium bicarbonate, potassium carbonate or the like may be used.

(2) The compounds as shown by formula (I), wherein A is methylene group, can be prepared by reducing the following compound (VII) which is prepared according to the preceding process of Item (1).

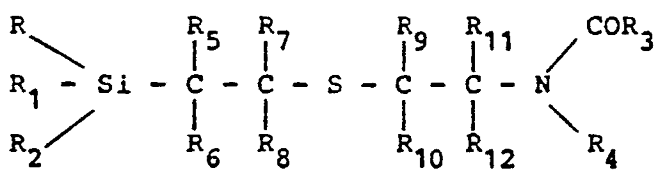

(VII)

wherein R, $R_1$, $R_2$, $R_3$, $R_4$, $R_5$, $R_6$, $R_7$, $R_8$, $R_9$, $R_{10}$, $R_{11}$ and $R_{12}$ have the meanings referred to above.
The reduction of carbonyl group in the compound can be carried out with use of a suitable reducing agent in a solvent. As the reducing agent, lithium aluminum hydride, sodium aluminum hydride, aluminum hydride, sodium borohydride, sodium borohydride-triethyloxonium tetrafluoroborate or the like may be used. As the solvent, ethyl ether, tetrahydrofuran, dioxane or the like ether; methanol, ethanol or the like alcohol may be used. The reaction temperature ranging from 0°C to the boiling point of the used solvent may be selected, by taking kinds of the raw material and solvent as well as other terms into consideration.

The starting compounds of formula (VII) can be also prepared by subjecting a compound represented by a formula

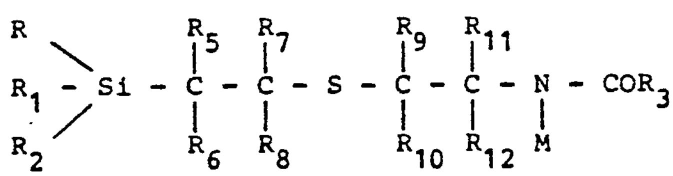

(VIII)

wherein R, $R_1$, $R_2$, $R_3$, $R_5$, $R_6$, $R_7$, $R_8$, $R_9$, $R_{10}$, $R_{11}$ and $R_{12}$ have the meanings referred to and M is sodium or potassium,

to a reaction with a compound represented by a formula

$$X—R_4 \qquad (IX)$$

wherein $R_4$ has the meaning as referred to and X is halogen, in same molar amount.

The compounds as shown by formula (VIII) can be prepared by subjecting the compound as shown by formula (VII), wherein $R_4$ is hydrogen, to a reaction in a solvent with sodium hydride, potassium hydride or the like base. As the solvent, ethyl ether, tetrahydrofuran, dioxane or the like ether may be used. It is most preferable to carry out the reaction at a temperature of from 0°C to the room temperature. The reaction of the compound (VIII) with the compound (IX) conveniently proceeds by using a catalytic amount of 18-crown-6 or the like crown ether and heating the reactants in the presence of a solvent. As the solvent, ethyl ether, tetrahydrofuran, dioxane or the like ether may be used. The reaction temperature may be determined according to the kinds of raw materials and solvent as well as other factors, but the boiling temperature of the used solvent is usually selected therefor.

(3) N-alkyl- and N-alkenyl derivatives can be prepared by removing the acyl group in the compounds (VII) through hydrolysis thereof. The hydrolyzing reaction conveniently proceeds by using a base in the presence of a solvent. As the base, potassium hydroxide, sodium hydroxide, potassium carbonate or the like may be used. Water or an alcohol such as methanol and ethanol are most preferable as the solvent. The reaction temperature may be determined according to the kind of raw material and solvent as well as other factors, but a temperature of from 20 to 40°C is usually selected therefor.

The N-alkyl and N-alkenyl derivatives obtained according to the method of Item (3) are liquids having a higher boiling point but they easily form a salt with hydrochloric acid, sulfuric acid, nitric acid or the like inorganic acid as well as acetic acid, succinic acid, maleic acid, fumaric acid, tartaric acid, citric acid or the like organic acid and deposit by crystallization. Therefore, the purity thereof can be made higher through a conventional recrystallization method.

(5) The compounds as shown by formula (I), wherein A is sulfonyl group, can be prepared by subjecting the compound as shown by formula (II) to a reaction with a compound represented by the formula

$$R_3—SO_2X \qquad (X)$$

wherein $R_3$ has the meaning defined above and X is halogen, in equi-molar amount. This reaction can be carried out under the same conditions as for the reaction of the compound (II) with the compound (V).

Each of the antitumor agents according to the invention develops its pharmaceutical action in either oral or non-oral dosage. A tablet, capsule or granules may be selected for the oral dosage. Depending on an intended form of the pharmaceutical agent, a conventional vehicle, grossing agent, coloring agent, flavoring agent, sweetening agent and the like may be added. For preparing an injection, a pH regulator, buffering agent, stabilizer, isotonics and the like may be added to the compound according to the invention. A suppository can be prepared in a conventional manner after adding the compound according to the invention and if necessary a surfactant to a usual vehicle.

A dosing amount of the antitumor agent for a patient can be determined according to symptoms, application form and other conditions, but in general, it is preferable to give in oral dosage 300 to 5000 mg/day for an adult. For administration by injection or suppository, it is preferable to give 25 to 500 mg and 100 to 2000 mg, respectively.

The invention will now be further illustrated by the following preparative Examples and pharmacological tests.

## Example 1

N-benzoyl-2-[[2-(trimethylsilyl)ethyl]thio]ethyl amine

To a solution of 4.43 g (25.0 mmol) of 2-[[2-(trimethylsilyl)ethyl]thio]ethyl amine and 2.53 g (25.0 mmol) of triethylamine dissolved in 50 ml of dichloromethane, 3.52 g (25.0 mmol) of benzoyl chloride is added slowly. The mixture is stirred at 25°C for 1 hour.

The reaction mixture is washed with water and dried over sodium sulfate and evaporated to dryness.

The resulting oily residue is distilled to yield N-benzoyl-2-[[2-(trimethylsilyl)ethyl]thio]ethyl amine (6.50 g, 92.5%).

b.p.: 230°C at 1 mm

| | | | |
|---|---|---|---|
| Anal. Calcd. for $C_{14}H_{23}NOSSi$: | C 59.74; | H 8.24; | N 4.98 |
| Found: | C 59.59; | H 8.18; | N 4.98 |

IR: $\nu_{max}^{neat}$ $cm^{-1}$:
3320 ($\nu_{NH}$), 2960, 2920 ($\nu_{CH}$), 1640 ($\nu_{C=O}$), 1603, 1490 ($\nu_{arom}$), 1535 ($\delta_{NH}$), 1250 ($\nu_{C-Si}$)

$^1H$—NMR ($CDCl_3$) δ:
0.01 (9H, s, $—Si(CH_3)_3$)

0.85 (2H, $A_2B_2$, ⪢$SiCH_2$—)
2.60 (2H, $A_2B_2$, ⪢$SiCH_2$*$CH_2$*S—)
2.77 (2H, t, J=6Hz, —*S$CH_2$*$CH_2$N—)
3.63 (2H, q, J=6Hz, —*$CH_2$*NHCO—)
6.87 (1H, m, —NHCO—)
7.3—7.9 (5H, m, Arom.H)
mass (EI/DI) m/e: 281 ($M^+$), 266 (−Me), 73 (base peak)

Example 2

N-(*p*-chlorobenzoyl)-2-[[2-(trimethylsilyl)ethyl]thio]ethyl amine

This compound is prepared by the similar procedure as in the case of Example 1, except for treatment with 4.38 g (25.0 mmol) of *p*-chlorobenzoyl chloride instead of benzoyl chloride.

Yield: 7.40 g (94.0%)
m.p.: 42—43°C

| | | | |
|---|---|---|---|
| Anal. Calcd. for $C_{14}H_{22}ClNOSSi$: | C 53.22; | H 7.02; | N 4.43 |
| Found: | C 52.94; | H 7.22; | N 4.42 |

IR: $\nu_{max}^{KBr}$ $cm^{-1}$:
3340 ($\nu_{NH}$), 2960, 2940, 2920 ($\nu_{CH}$), 1635 ($\nu_{C=O}$), 1490 ($\nu_{arom}$), 1545 ($\delta_{NH}$), 1490 ($\nu_{C=C}$), 1250 ($\nu_{C-Si}$)
$^1$H—NMR ($CDCl_3$) δ:
0.01 (9H, s, —$Si(CH_3)_3$)
0.85 (2H, $A_2B_2$, ⪢Si*$CH_2$*—)
2.60 (2H, $A_2B_2$, ⪢$SiCH_2$*$CH_2$*S—)
2.77 (2H, t, J=6Hz, —*S$CH_2$*$CH_2$N—)
3.63 (2H, q, J=6Hz, —*$CH_2$*NHCO—)
6.67 (1H, m, —NHCO—)
7.36 (2H, AA'XX', arom.H)
7.73 (2H, AA'XX', arom.H)
mass (EI/DI) m/e: 315 ($M^+$), 317 (M+2), 73 (base peak)

Example 3

N-(1-naphthoyl)-2-[[2-(trimethylsilyl)ethyl]thio]ethyl amine

This compound is prepared by the similar procedure as in the case of Example 1, except for treatment with 4.76 g (25.0 mmol) of 1 naphthoyl chloride instead of benzoyl chloride.

Yield: 7.70 g (93.1%)
m.p.: 45—46°C

| | | | |
|---|---|---|---|
| Anal. Calcd. for $C_{18}H_{25}NOSSi$: | C 65.21; | H 7.60; | N 4.22 |
| Found: | C 65.46; | H 7.76; | N 4.46 |

IR: $\nu_{max}^{KBr}$ $cm^{-1}$:
3340 ($\nu_{NH}$), 2960, 2920 ($\nu_{C-H}$), 1640 ($\nu_{C=O}$), 1540 ($\delta_{NH}$), 1250 ($\nu_{C-Si}$)
$^1$H—NMR ($CDCl_3$) δ:
0.01 (9H, s, —$Si(CH_3)_3$)
0.85 (2H, $A_2B_2$, ⪢$SiCH_2$—)
2.63 (2H, $A_2B_2$, ⪢$SiCH_2$*$CH_2$*S—)
2.83 (2H, t, J=6Hz, —*S$CH_2$*$CH_2$N—)
3.62 (2H, q, J=6Hz, —*$CH_2$*NHCO—)
6.50 (1H, m, —NHCO—)
7.3—8.5 (7H, m, arom.H)
mass (EI/DI) m/e: 331 ($M^+$), 73 (base peak)

Example 4

N-nicotinoyl-2-[[2-(trimethylsilyl)ethyl]thio]ethyl amine

This compound is prepared by the similar procedure as in the case of Example 1, except for treatment with 4.45 g (25.0 mmol) of nicotinic acid chloride hydrochloride instead of benzoyl chloride and 5.05 g (50.0 mmol) of triethylamine.

Yield: 6.50 g (92.2%)
b.p.: 248°C (1 mm)

| | | | |
|---|---|---|---|
| Anal. Calcd. for $C_{13}H_{22}N_2OSSi$: | C 55.28; | H 7.85; | N 9.92 |
| Found: | C 54.81; | H 8.14; | N 9.79 |

IR: $\nu_{max}^{neat}$ $cm^{-1}$:
3320 ($\nu_{NH}$), 2960, 2920 ($\nu_{CH}$), 1655 ($\nu_{C=O}$), 1595 ($\nu_{C=C}$), 1545 ($\delta_{NH}$), 1480 ($\nu_{C=C}$), 1250 ($\nu_{C-Si}$)
$^1$H—NMR ($CDCl_3$) δ:
0.01 (9H, s, —$Si(CH_3)_3$)
0.85 (2H, $A_2B_2$, ⪢Si*$CH_2$*—)

2.60 (2H, $A_2B_2$, $\gtrdot SiCH_2\mathit{CH_2}S$—)
2.80 (2H, t, J=6Hz, —$S\mathit{CH_2}CH_2N$—)
3.65 (2H, q, J=6Hz, —$\mathit{CH_2}NHCO$—)
7.05 (1H, m, —NHCO—)
7.34 (1H, dd, J=5Hz, 8Hz,

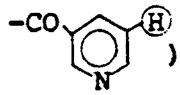
)

8.10 (1H, dt, J=2Hz, 8Hz,

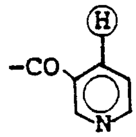
)

8.67 (1H, dd, J=2Hz 5Hz,

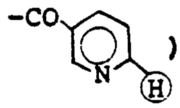
)

8.97 (1H, d, J=2Hz,

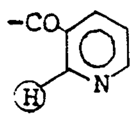
)

mass (EI/DI) m/e: 282 ($M^+$), 73 (base peak)

Example 5

N-(2-furoyl)-2-[[2-(trimethylsilyl)ethyl]thio]ethyl amine

This compound is prepared by the similar procedure as in the case of Example 1, except for treatment with 3.26 g (25.0 mmol) of 2-furoyl chloride instead of benzoyl chloride.

Yield: 6.20 g (91.5%)
b.p.: 225°C (1 mm)

| | | | |
|---|---|---|---|
| Anal. Calcd. for $C_{12}H_{21}NO_2SSi$: | C 53.10; | H 7.80; | N 5.16 |
| Found: | C 52.55; | H 8.04; | N 5.13 |

IR: $\nu_{max}^{neat}$ $cm^{-1}$:
3320 ($\nu_{NH}$), 2960, 2920 ($\nu_{CH}$), 1650 ($\nu_{C=O}$), 1595, 1575 ($\nu_{C=C}$), 1530 ($\delta_{NH}$), 1480 ($\nu_{C=C}$), 1250 ($\nu_{C-Si}$)

$^1H$—NMR ($CDCl_3$) δ:
0.01 (9H, s, —$Si(CH_3)_3$)
0.85 (2H, $A_2B_2$, $\gtrdot Si\mathit{CH_2}$—)
2.60 (2H, $A_2B_2$, $\gtrdot SiCH_2\mathit{CH_2}S$—)
2.77 (2H, t, J=6Hz, —$S\mathit{CH_2}CH_2N$—)
3.61 (2H, q, J=6Hz, —$\mathit{CH_2}NHCO$—)
6.46 (1H, dd, J=2Hz, 3.6Hz,

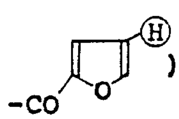
)

6.82 (1H, m, —NHCO—)
7.10 (1H, d, J=3.6Hz,

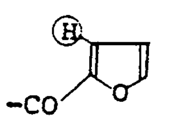
)

7.42 (1H, m,

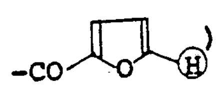
)

mass (EI/DI) m/e: 271 ($M^+$), 73 (base peak)

### Example 6

N-thenoyl-2-[[2-(trimethylsilyl)ethyl]thio]ethyl amine

This compound is prepared by the similar procedure as in the case of Example 1, except for treatment with 3.67 g (25.0 mmol) of thenoyl chloride instead of benzoyl chloride.

Yield: 6.60 g (92.1%)

b.p.: 230°C (1 mm)

| | | | |
|---|---|---|---|
| Anal. Calcd. for $C_{12}H_{21}NOS_2Si$: | C 50.13; | H 7.36; | N 4.87 |
| Found: | C 49.35; | H 7.55; | N 4.81 |

IR: $\nu_{max}^{neat}$ $cm^{-1}$:

3320 ($\nu_{NH}$), 2960, 2920 ($\nu_{CH}$), 1630 ($\nu_{C=O}$), 1550 ($\delta_{NH}$), 1515 ($\nu_{C=C}$), 1420 ($\nu_{C=C}$), 1250 ($\nu_{C-Si}$)

$^1H$—NMR ($CDCl_3$) δ:

0.01 (9H, s, $-Si(CH_3)_3$)
0.85 (2H, $A_2B_2$, $\gg Si\mathit{CH_2}-$)
2.60 (2H, $A_2B_2$, $\gg SiCH_2\mathit{CH_2}S-$)
2.77 (2H, t, J=6Hz, $-S\mathit{CH_2}CH_2N-$)
3.61 (2H, q, J=6Hz, $-\mathit{CH_2}NHCO-$)
6.70 (1H, m, —NHCO—)
7.03 (1H, m,

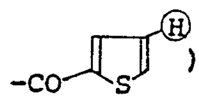 )

7.44 (1H, m,

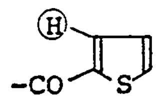 )

7.50 (1H, dd, J=1Hz,

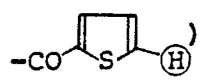 )

mass (EI/DI) m/e: 287 ($M^+$), 73 (base peak)

### Example 7

N-acetyl-2-[[2-(trimethylsilyl)ethyl]thio]ethyl amine

This compound is prepared by the similar procedure as in the case of Example 1, except for treatment with 1.97 g (25.0 mmol) of acetyl chloride instead of benzoyl chloride.

Yield: 5.08 g (92.8%)

b.p.: 187°C (1 mm)

| | | | |
|---|---|---|---|
| Anal. Calcd. for $C_9H_{21}NOSSi$: | C 49.27; | H 9.65; | N 6.38 |
| Found: | C 48.67; | H 9.81; | N 6.55 |

IR: $\nu_{max}^{CHCl_3}$ $cm^{-1}$:

3460 ($\nu_{NH}$), 3010, 2970 ($\nu_{CH}$), 1670 ($\nu_{C=O}$), 1515 ($\delta_{NH}$), 1215 ($\nu_{C-Si}$)

$^1H$—NMR ($CDCl_3$) δ:

0.01 (9H, s, $-Si(CH_3)_3$)
0.85 (2H, $A_2B_2$, $\gg Si\mathit{CH_2}-$)
1.97 (3H, s, $\mathit{CH_3}CO-$)
2.55 (2H, $A_2B_2$, $\gg SiCH_2\mathit{CH_2}S-$)
2.65 (2H, t, J=6Hz, $-SCH_2CH_2N-$)
3.41 (2H, q, J=6Hz, $-CH_2NHCO-$)
6.12 (1H, m, —NHCO—)

mass (EI/DI) m/e: 219 ($M^+$), 73 (base peak)

### Example 8

N-cyclohexanecarbonyl-2-[[2-(trimethylsilyl)ethyl]thio]ethyl amine

This compound is prepared by the similar procedure as in the case of Example 1, except for treatment with 3.67 g (25.0 mmol) of cyclohexanecarboxylic acid chloride instead of benzoyl chloride.

Yield: 6.50 g (90.6%)

b.p.: 240°C (1 mm)

| | | | |
|---|---|---|---|
| Anal. Calcd. for $C_{14}H_{29}NOSSi$: | C 58.48; | H 10.17; | N 4.87 |
| Found: | C 57.97; | H 10.39; | N 5.09 |

IR: $\nu_{max}^{neat}$ $cm^{-1}$:
3320 ($\nu_{NH}$), 2940, 2860 ($\nu_{CH}$), 1645 ($\nu_{C=O}$), 1545 ($\delta_{NH}$), 1250 ($\nu_{C-Si}$)

$^1H$—NMR ($CDCl_3$) δ:
0.01 (9H, s, —$Si(CH_3)_3$)
0.84 (2H, $A_2B_2$, ⩾$SiCH_2$—)
1.0—2.3 (11Hz, m, H of cyclohexyl)
2.55 (2H, $A_2B_2$, ⩾$Si\textit{CH}_2\textit{CH}_2S$—)
2.63 (2H, t, J=6Hz, —$S\textit{CH}_2CH_2N$—)
3.41 (2H, q, J=6Hz, —$\textit{CH}_2NHCO$—)
5.97 (1H, m, —NHCO—)

mass (EI/DI) m/e: 287 ($M^+$), 73 (base peak)

## Example 9

N-octanoyl-2-[[2-(trimethylsilyl)ethyl]thio]ethyl amine

This compound is prepared by the similar procedure as in the case of Example 1, except for treatment with 4.07 g (25.0 mmol) of octanoyl chloride instead of benzoyl chloride.

Yield: 6.80 g (89.8%)

b.p.: 228°C (1 mm)

| | | | |
|---|---|---|---|
| Anal. Calcd. for $C_{15}H_{33}NOSSi$: | C 59.35; | H 10.96; | N 4.61 |
| Found: | C 58.97; | H 11.21; | N 4.62 |

IR: $\nu_{max}^{CHCl_3}$ $cm^{-1}$:
3460 ($\nu_{NH}$), 2960, 2940, 2860 ($\nu_{CH}$), 1665 ($\nu_{C=O}$), 1510 ($\delta_{NH}$), 1250 ($\nu_{C-Si}$)

$^1H$—NMR ($CDCl_3$) δ:
0.01 (9H, s, —$Si(CH_3)_3$)
0.84 (2H, $A_2B_2$, ⩾$Si\textit{CH}_2$—)
0.85 (3H, t, J=6.5Hz, —$(CH_2)_6\textit{CH}_3$)
1.0—1.8 (10H, m)
2.17 (2H, t, J=6.5Hz, —NHCO—$\textit{CH}_2$—)
2.55 (2H, $A_2B_2$, ⩾$SiCH_2\textit{CH}_2S$—)
2.65 (2H, t, J=6Hz, —$S\textit{CH}_2CH_2N$—)
3.41 (2H, q, J=6Hz, —$\textit{CH}_2NHCO$—)
5.96 (1H, m, —NHCO—)

mass (EI/DI) m/e: 303 ($M^+$), 73 (base peak)

## Example 10

N-(*p*-toluenesulfonyl)-2-[[2-(trimethylsilyl)ethyl]thio]ethyl amine

This compound is prepared by the similar procedure as in the case of Example 1, except for treatment with 4.77 g (25.0 mmol) of *p*-toluenesulfonyl chloride instead of benzoyl chloride.

Yield: 7.70 g (93.1%)

b.p.: 279°C (1 mm)

| | | | |
|---|---|---|---|
| Anal. Calcd. for $C_{14}H_{25}NO_2SSi$: | C 50.71; | H 7.60; | N 4.22 |
| Found: | C 50.28; | H 7.80; | N 4.34 |

IR: $\nu_{max}^{neat}$ $cm^{-1}$:
3300 ($\nu_{NH}$), 2960, 2920 ($\nu_{CH}$), 1600 ($\nu_{arom.}$), 1500 ($\nu_{arom.}$), 1415 ($\nu_{SO_2}$), 1250 ($\nu_{C-Si}$), 1160 ($\nu_{SO_2}$)

$^1H$—NMR ($CDCl_3$) δ:
0.01 (9H, s, —$Si(CH_3)_3$)
0.78 (2H, $A_2B_2$, ⩾$Si\textit{CH}_2$—)
2.42 (2H, $A_2B_2$, ⩾$SiCH_2\textit{CH}_2S$—)
2.43 (3H, s, arom.$CH_3$)
2.60 (2H, t, J=6Hz, —$S\textit{CH}_2CH_2N$—)
3.11 (2H, q, J=6Hz, —$\textit{CH}_2NHSO_2$—)
5.13 (1H, t, J=6Hz, —$N\textit{H}SO_2$—)
7.30 (2H, d, J=8Hz, arom.H)
7.76 (2H, d, J=8Hz, arom.H)

mass (EI/DI) m/e: 331 ($M^+$), (73 base peak)

## Example 11

N-methanesulfonyl-2-[[2-(trimethylsilyl)ethyl]thio]ethyl amine

This compound is prepared by the similar procedure as in the case of Example 1, except for treatment with 2.85 g (25.0 mmol) of methanesulfonyl chloride instead of benzoyl chloride.

Yield: 5.77 g (90.5%)

m.p.: 41—41°C

Anal. Calcd. for $C_8H_{21}NO_2S_2Si$: C 37.61; H 8.29; N 5.48
Found: C 37.30; H 8.55; N 5.48
IR: $\nu_{max}^{KBr}$ $cm^{-1}$:
3280 ($\nu_{NH}$), 1410, 1150 ($\nu_{SO_2}$), 1250 ($\nu_{C-Si}$)
$^1H$—NMR ($CDCl_3$) δ:
0.00 (9H, s, —$Si(CH_3)_3$)
0.81 (2H, $A_2B_2$, >Si*CH₂*—)
2.52 (2H, $A_2B_2$, >$SiCH_2$*CH₂*S—)
2.68 (2H, t, J=6Hz, —S*CH₂*$CH_2$N—)
2.93 (3H, s, —$NHSO_2$*CH₃*)
3.25 (2H, q, J=6Hz, —$SCH_2$*CH₂*N—)
5.03 (1H, t, J=6Hz, —N*H*$SO_2$—)
mass (EI/DI) m/e: 255 ($M^+$), 73 (base peak)

## Example 12

N-formyl-2-[[2-(trimethylsilyl)ethyl]thio]ethyl amine

To a solution of 0.500 g (2.80 mmol) of 2-[[2-(trimethylsilyl)ethyl]thio]ethyl amine in 6.30 g (0.140 mol) of 98% formic acid, 2.10 g (20.0 mmol) of acetic acid is added dropwise at 0—5°C.

The mixture is stirred at 0—5°C for 30 min, and then at 25°C for 1 hr.

The reaction mixture is neutralized with a dilute aqueous ammonia, and extracted with diethyl ether, the extract is washed with water, dried over sodium sulfate, and then evaporated in vacuo.

The resulting oily residue is distilled to afford N-formyl-2-[[2-(trimethylsilyl)ethyl]thio]ethyl amine (0.550 g, 95.0%).

IR: $\nu_{max}^{CHCl_3}$ ($cm^{-1}$):
3455 ($\nu_{NH}$), 2970 ($\nu_{CH}$), 1690 ($\nu_{C=O}$), 1505 ($\delta_{NH}$), 1250 ($\nu_{C-Si}$)
$^1H$—NMR ($CDCl_3$) δ:
0.01 (9H, s, $(CH_3)_3Si$—)
0.83 (2H, $A_2B_2$, >Si*CH₂*$CH_2$S—)
2.56 (2H, $A_2B_2$, >$SiCH_2$*CH₂*S—)
2.70 (2H, t, J=6Hz, —S*CH₂*$CH_2$NH—)
3.52 (2H, q, J=6Hz, —*CH₂*NHCO—)
8.17 (1H, brs, —NHCO—)
mass (EI/DI) m/e: 205 ($M^+$), 73 (base peak)

## Example 13

N-trifluoroacetyl-2-[[2-(trimethylsilyl)ethyl]thio]ethyl amine

To a solution of 3.54 g (20.0 mmol) of 2-[[2-(trimethylsilyl)ethyl]thio]ethyl amine in 10 ml of absolute pyridine, 8.40 g (40.0 mmol) of trifluoroacetic anhydride is added dropwise under argon at 0°C, and then stirred for 7 hrs at 20°C.

The reaction mixture is poured into water, neutralized with concentrated hydrochloric acid, and extracted with diethyl ether. The combined organic layers are washed with water, dried over sodium sulfate, and then evaporated in vacuo. The residue is distilled to afford N-trifluoroacetyl-2-[[2-(trimethylsilyl)ethyl]thio]ethyl amine (5.00 g, 90.9%).

b.p.: 170°C (2 mm)
Anal. Calcd. for $C_9H_{18}F_3NOSSi$: C 39.54; H 6.64; N 5.12
Found: C 39.26; H 6.85; N 5.29
IR: $\nu_{max}^{CHCl_3}$ ($cm^{-1}$):
3460 ($\nu_{NH}$), 2980 ($\nu_{CH}$), 1730 ($\nu_{C=O}$), 1540 ($\delta_{NH}$), 1260 ($\nu_{C-Si}$), 1170 ($\nu_{C-F}$)
$^1H$—NMR ($CDCl_3$) δ:
0.01 (9H, s, $(CH_3)_3Si$—)
0.82 (2H, $A_2B_2$, >Si*CH₂*$CH_2$S—)
2.55 (2H, $A_2B_2$, >$SiCH_2$*CH₂*S—)
2.71 (2H, t, J=6Hz, —S*CH₂*$CH_2$N—)
3.52 (2H, q, J=6Hz, —*CH₂*NHCO—)
6.88 (1H, brs, —NHCO—)
mass (EI/DI) m/e: 273 ($M^+$), 73 (base peak)

## Example 14

N-allyl-N-trifluoroacetyl-2-[[2-(trimethylsilyl)ethyl]thio]ethyl amine

115 mg (4.80 mmol) of sodium hydride, freed from protective mineral oil by three 3-ml *n*-hexane washings, is added to 6 ml of absolute tetrahydrofuran under argon at 0—5°C. The addition of 1.10 g (4.00 mmol) of N-trifluoroacetyl-2-[[2-(trimethylsilyl)ethyl]thio]ethyl amine to the stirred suspension is attended by vigorous gas ($H_2$) evolution. After 5 min, 5 mg of 18-crown-6 and 678 mg (5.60 mmol) of freshly distilled allyl bromide are added, and then at reflux temperature for 12 hrs.

Tetrahydrofuran is removed by evaporation in vacuo, the residue is dissolved in diethyl ether, washed

with a dilute hydrochloric acid solution and water, the organic layer is dried over sodium sulfate, and then evaporated in vacuo.

The oily residue is distilled to afford N-allyl-N-trifluoroacetyl-2-[[2-(trimethylsilyl)ethyl]thio]ethyl amine (1.10 g, 87.3%).

b.p.: 140°C (1 mm)

Anal. Calcd. for $C_{12}H_{22}F_3NOSSi$: C 45.98; H 7.07; N 4.47
Found: C 46.23; H 7.34; N 4.62

IR: $\nu_{max}^{CHCl_3}$ ($cm^{-1}$):
2960 ($\nu_{NH}$), 1690 ($\nu_{C=O}$), 1250 ($\nu_{C-Si}$), 1150 ($\nu_{C-F}$)

$^1H$—NMR ($CDCl_3$) δ:
0.01 (9H, s, $(CH_3)_3Si$—)
0.84 (2H, $A_2B_2$, $\gtrdot Si\mathit{CH_2}CH_2S$—)
2.62 (4H, m, —$CH_2SCH_2$—)
3.53 (2H, t, J=6Hz, —$SCH_2\mathit{CH_2}N$—)
4.05 (2H, d, J=6Hz, —$N\mathit{CH_2}CH=CH_2$)
5.0—5.3 (2H, m, —$CH_2CH=\mathit{CH_2}$)
5.6—6.0 (1H, m, —$CH_2\mathit{CH}=CH_2$)
mass (EI/DI) m/e: 313 ($M^+$)

The following compounds were used in pharmaceutical tests; SKK—009 to 011 are maleate salts of compounds known from EP—0046242 and the data is given for comparison. SKK—012 to SKK—029 correspond to the products of Examples 1 to 11, as follows:

| | | | |
|---|---|---|---|
| SKK—012 | Ex 1 | SKK—026 | Ex 8 |
| SKK—020 | Ex 2 | SKK—027 | Ex 9 |
| SKK—021 | Ex 4 | SKK—028 | Ex 10 |
| SKK—022 | Ex 5 | SKK—029 | Ex 11 |
| SKK—023 | Ex 6 | | |
| SKK—024 | Ex 3 | | |
| SKK—025 | Ex 7 | | |

| Compd. No. | Formula |
|---|---|
| SKK-009 | 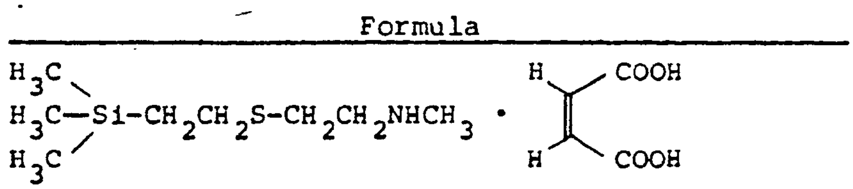 |
| SKK-010 | 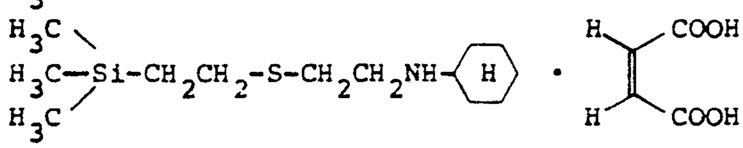 |
| SKK-011 | 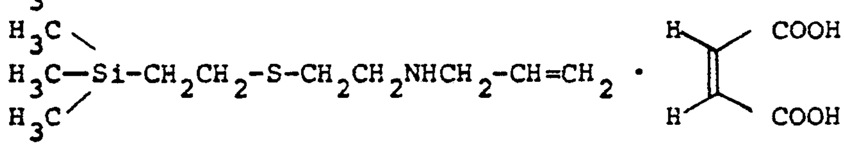 |
| SKK-012 | 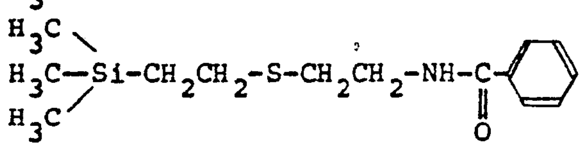 |
| SKK-020 | 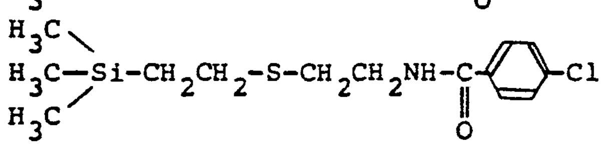 |
| SKK-021 | 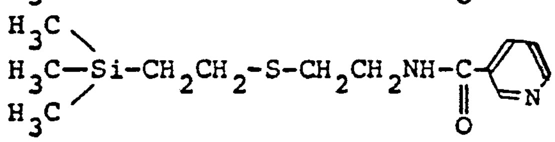 |
| SKK-022 | 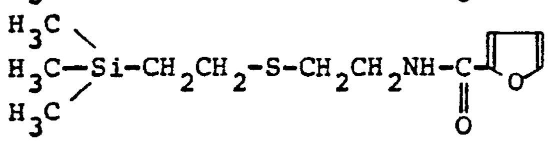 |

| Compd. No. | Formula |
|---|---|
| SKK-023 | 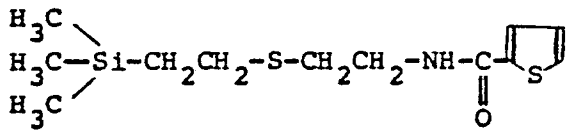 |
| SKK-024 | 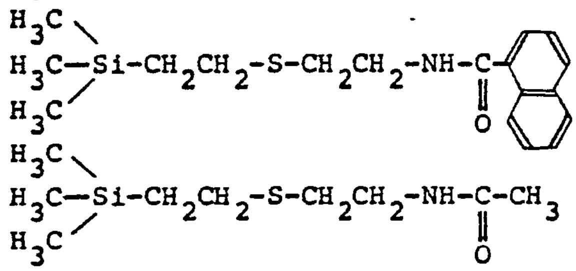 |
| SKK-025 | |
| SKK-026 | 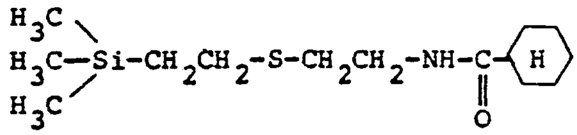 |
| SKK-027 | 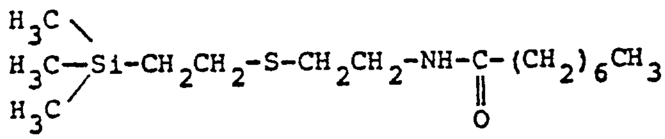 |
| SKK-028 | 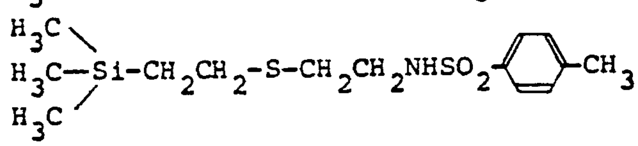 |
| SKK-029 | 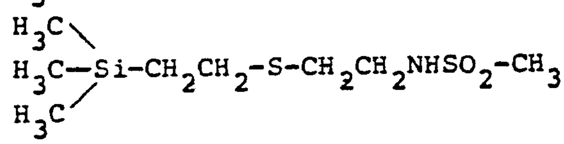 |

Tests on Pharmaceutical Effects

*Test 1* (proliferation inhibiting effect in explantation)

The following carcinomata are employed.

1) Lewis lung carcinoma (hereinafter referred to as "LLC"),
2) Ehrlich ascites carcinoma (hereinafter referred to as "EAC"),
3) K. B. carcinoma (hereinafter referred to as "KB"), and
4) B—16 melanoma carcinoma (hereinafter referred to as "B—16").

$1 \times 10^5$ cells of each carcinoma were cultured for 24 hours in an ox serum eagle culture solution with use of a culture test tube and a carbon dioxide gas culturing device (5% $CO_2$, 37°C). After 24 hours, the used culture solution was flowed-out and each 0.1 ml of a freshly prepared culture solution containing a compound according to the invention was added to further continue the culture for 3 days. After completion of the culture, number of living cells was measured with use of a dye-exclusion method using tripan blue dying. To each of control groups, 0.1 ml of the same culture solution but containing no compound according to the invention was added.

Concentrations at 50% inhibition of the carcinoma cells ($IC_{50}$) were determined between the testing groups and control groups, according to the manner stated above. Results are shown in following Table.

| Compound No. | $IC_{50}$ (µg/ml) | | | |
| --- | --- | --- | --- | --- |
| | LLC | EAC | KB | B-16 |
| SKK-012 | 4.3 | 15 | 17 | 16 |
| SKK-021 | 5 | 12 | 10 | <5 |
| SKK-022 | 9 | 15 | 17 | 20 |
| SKK-024 | 13 | 18 | 14 | 25 |
| SKK-028 | 14 | 30 | 10 | 14 |

*Test 2* (carcinoma cell weight inhibiting effect in mice)

B—16 and EAC were given to BDF and ICR—CD mice, respectively by a hypodermic injection. $1.0 \times 10^5$ cells on B—16 and $5.0 \times 10^6$ cells on EAC were given to each mouse. Each of the compounds according to the invention was orally dosed for 5 days in an amount of 1/5 $LD_{50}$ (one time/day). After lapsed 21 days from the final dosage, the carcinoma of each mouse was exenterated, its weight measured and a mean weight calculated as the inhibiting ratio which is shown in following Table.

| Compound No. | Name of Carcinomata | |
| --- | --- | --- |
| | B-16 | EAC |
| SKK-012 | 62 | 76 (inhibiting ratio) |
| SKK-021 | 93 | 70 |
| SKK-022 | 58 | 65 |
| SKK-024 | 60 | 62 |
| SKK-028 | 85 | 55 |

*Test 3* (acute toxicity)

Each of compounds according to the invention was suspended in 0.1% P—1570 aqueous solution of sugar ester or 0.5% CMC aqueous solution. The suspension was abdominally or orally dosed to ICR female mice (weight: 20 ± 1 g). The tested mice were observed for 7 days and $LD_{50}$ was determined based on Lichfield-Wilcoxon method. Results were shown in following Table.

| Compound No. | Acute Toxicity ($LD_{50}$, mg/kg) | |
|---|---|---|
| | Abdominal Dosage | Oral Dosage |
| SKK-009 | 189 | 800 |
| SKK-010 | 152 | 825 |
| SKK-011 | 134 | 846 |
| SKK-012 | 1190 | >2000 |
| SKK-021 | <500 | >2000 |
| SKK-022 | < 1000 | >2000 |
| SKK-023 | > 1000 | >2000 |
| SKK-024 | > 2000 | >3000 |
| SKK-025 | ±100 | 1000 - 2000 |
| SKK-026 | 500 - 1000 | 1000 - 2000 |
| SKK-027 | 2000 - 3000 | >3000 |
| SKK-028 | > 2500 | >3000 |

Examples for the Preparation of Medicines

Example 1 (tablet)

Tablets were prepared in a conventional manner with use of following components.

| | |
|---|---|
| Compound (SKK—012) | 100 (mg) |
| Crystallized cellulose | 20 |
| Lactose | 41 |
| Corn starch | 30 |
| Hydroxypropyl cellulose | 6 |
| Magnesium stearate | 3 |
| | 200 mg/tablet |

Example 2 (capsule)

Following components were mixed and sealed in a capsule in a conventional manner.

| | |
|---|---|
| Compound (SKK—022) | 200 (mg) |
| Crystallized cellulose | 50 |
| Silicic anhydride | 2 |
| Magnesium stearate | 3 |
| | 255 mg/capsule |

Example 3 (granules)

Granules were prepared in a conventional manner with use of following components and individually packed in aluminum foil bag.

| | |
|---|---|
| Compound (SKK—021) | 500 (mg) |
| Lactose | 323 |
| Corn starch | 150 |
| Polyvinyl pyrrolidone | 25 |
| Silicic anhydride | 2 |
| | 1000 mg/package |

Example 4 (suppository)

Suppositories were prepared by mixing following components and moulding the same.

| | |
|---|---|
| Compound (SKK—028) | 300 (mg) |
| Witepsol W—35 (triglycerides of saturated vegetable fatty acids*) | 1700 |
| | 2000 mg/suppository |

Example 5 (injectable solution)

A solution for injectional dosage was prepared by mixing following components and was sealed in a glass ampule in a conventional manner.

| | |
|---|---|
| Compound (SKK—009) | 25 (mg) |
| Sodium chloride | 25 |
| Distilled water for injection | suitable amount |
| | 10 ml/ampule |

**Claims**

1. An organosilicon compound represented by the formula

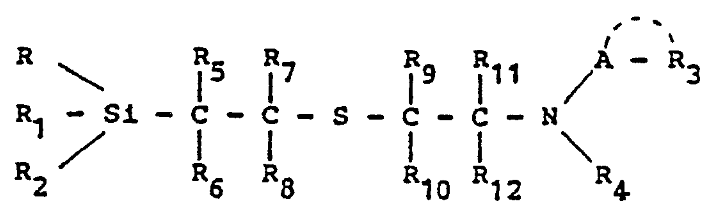

(I)

wherein

A represents a carbonyl or sulfonyl group;

R, $R_1$ and $R_2$ each represents an alkyl group;

$R_3$ represents an alkyl, trifluoroalkyl, alkenyl, phenyl, p-chlorophenyl, p-methylphenyl, naphthyl, pyridyl or furyl or thienyl group;

$R_4$ represents a hydrogen atom, an alkyl or alkenyl group;

$R_5$, $R_6$, $R_7$, $R_8$, $R_9$, $R_{10}$, $R_{11}$ and $R_{12}$ each represents a hydrogen atom or an alkyl group;

or a non-toxic salt thereof.

2. An organosilicon compound as claimed in Claim 1, wherein R, $R_1$ and $R_2$ are each a methyl group, and $R_5$, $R_6$, $R_7$, $R_8$, $R_9$, $R_{10}$, $R_{11}$ and $R_{12}$ are each a hydrogen atom.

3. An organosilicon compound as claimed in Claim 1 or 2, wherein —$R_3$ is an alkenyl group.

4. A maleate of an organosilicon compound (I) as claimed in Claim 1.

5. A process for the preparation of an organosilicon compound or salt thereof as defined in Claim 1, wherein A is a carbonyl group, which comprises reacting a compound represented by the formula:

* "Witepsol" is a registered Trade Mark.

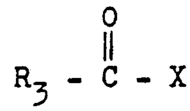 or 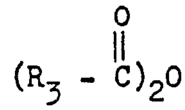

wherein X is halogen and $R_3$ is as defined in Claim 1 or 2, with a compound represented by the formula:

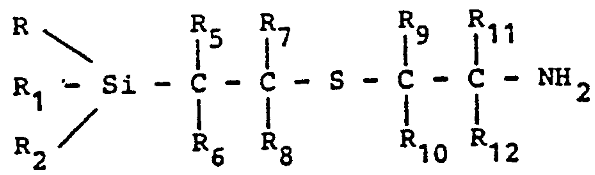 (II)

wherein R, $R_1$, $R_2$, $R_5$, $R_6$, $R_7$, $R_8$, $R_9$, $R_{10}$, $R_{11}$ and $R_{12}$ are as defined in Claim 1 or 2, and if necessary, converting the resulting compound into the non-toxic salt by reaction with an organic acid.

6. A process for the preparation of an organosilicon compound or salt thereof as defined in Claim 1, wherein A is a sulfonyl group, which comprises reacting a compound (II) as defined in Claim 5 with a sulfonyl compound represented by the formula:

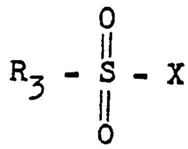

wherein X is a halogen atom and $R_3$ is as defined in Claim 1, and if necessary, converting the resulting compound into the non-toxic salt by reaction with an organic acid.

7. A process for the preparation of an organosilicon compound or salt thereof as defined in any of Claims 1, 2 or 4, wherein —$R_3$ is —$CH_3$, which comprises treating a compound (II) as defined in Claim 6 with formic acid and acetic anhydride, reducing the formyl product and if necessary, converting the resulting compound into the non-toxic salt.

8. A process for the preparation of an organosilicon compound (I) as defined in Claim 1, 2 or 4, wherein —A—$R_3$ is methyl and $R_4$ is hydrogen, which comprises subjecting a compound represented by the formula:

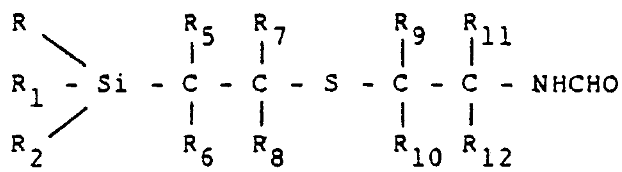

wherein R, $R_1$, $R_2$, $R_5$, $R_6$, $R_7$, $R_8$, $R_9$, $R_{10}$, $R_{11}$ and $R_{12}$ are as defined in Claim 1 or 2, to reduction, and, if necessary, converting the resulting compound into the non-toxic salt by reaction with an organic acid.

9. A process for the preparation of an organosilicon compound or salt thereof as claimed in Claim 1, 2 or 4, wherein A is carbonyl, which comprises subjecting a compound represented by the formula

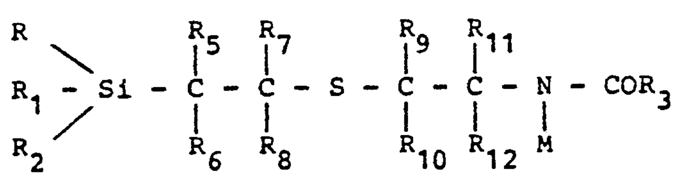 (VIII)

wherein R, $R_1$, $R_2$, $R_3$, $R_5$, $R_6$, $R_7$, $R_8$, $R_9$, $R_{10}$, $R_{11}$ and $R_{12}$ are as defined in Claim 1 or 2, to reaction with sodium or potassium hydroxide, subjecting the resulting compound, wherein the N—H is N—Na or N—K, to reaction with a compound represented by the formula

$R_4$—X

wherein X is halogen and $R_4$ is an alkyl or alkenyl group, and, if necessary, converting the resulting compound into the non-toxic salt by reaction with an organic acid.

10. An anti-tumor agent comprising as an effective component an organosilicon compound or salt as defined in any of Claims 1 to 4.

11. A process as claimed in Claim 5 or 6, wherein $R_4$ is a hydrogen atom.
12. A process as claimed in Claim 5, wherein $R_3$ is an alkyl or substituted alkyl group.
13. A process as claimed in Claim 6, wherein $R_3$ is an alkyl, phenyl or substituted phenyl group.

**Patentansprüche**

1. Eine Organosiliziumverbindung, dargestellt durch die Formel

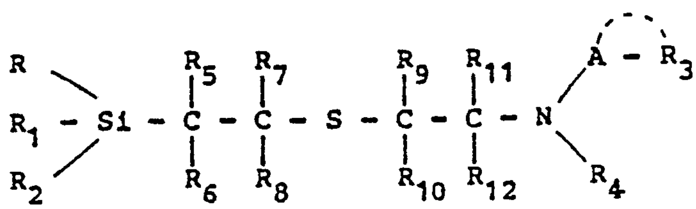

(I)

worin
A eine Carbonyl- oder Sulfonylgruppe bedeutet;
R, $R_1$ und $R_2$ jewils eine Alkylgruppe bedeuten;
$R_3$ eine Alkyl-, Trifluoralkyl-, Alkenyl-, Phenyl-, p-Chlorphenyl-, p-Methylphenyl-, Naphthyl-, Pyridyl- oder Furyl- oder Thienylgruppe bedeutet;
$R_4$ eine Wasserstoffatom, eine Alkylgruppe oder eine Alkenylgruppe bedeutet;
$R_5$, $R_6$, $R_7$, $R_8$, $R_9$, $R_{10}$, $R_{11}$ und $R_{12}$ jeweils ein Wasserstoffatom oder eine Alkylgruppe bedeuten;
oder eine nichttoxisches Salz davon.

2. Eine Organosiliziumverbindung nach Anspruch 1, worin R, $R_1$ und $R_2$ jeweils eine Methylgruppe, und $R_5$, $R_6$, $R_7$, $R_8$, $R_9$, $R_{10}$, $R_{11}$ und $R_{12}$ jeweils ein Wasserstoffatom bedeuten.
3. Eine Organosiliziumverbindung nach Anspruch 1 oder 2, worin —$R_3$ eine Alkenylgruppe ist.
4. Ein Maleat einer Organosiliziumverbindung (I) nach Anspruch 1.
5. Ein Verfahren zur Herstellung einer Organosiliziumverbindung oder eines Salzes davon nach Anspruch 1, worin A eine Carbonylgruppe ist, darin bestehend, daß man eine Verbindung, dargestellt durch die Formel

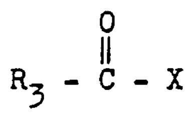 oder 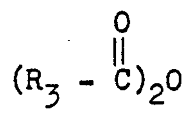

worin X Halogen ist und $R_3$ die in den Ansprüchen 1 oder 2 genannte Bedeutung hat, mit einer Verbindung, dargestellt durch die Formel

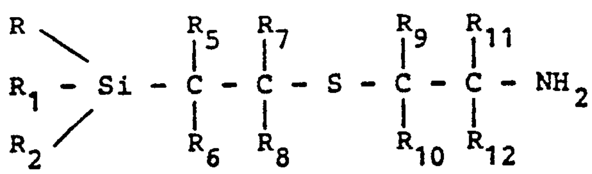

(II)

worin R, $R_1$, $R_2$, $R_5$, $R_6$, $R_7$, $R_8$, $R_9$, $R_{10}$, $R_{11}$ und $R_{12}$ die in den Ansprüchen 1 oder 2 angegebenen Bedeutung haben, reagiert und gegebenenfalls die erhaltene Verbindung durch Reaktion mit einer organischen Säure in das nichttoxische Salz überführt wird.

6. Ein Verfahren zur Herstellung einer Organosiliziumverbindung oder eines Salzes nach Anspruch 1, worin A eine Sulfonylgruppe ist, darin bestehend, daß man eine Verbindung (II) nach Anspruch 5 mit einer Sulfonylverbindung, dargestellt durch die Formel:

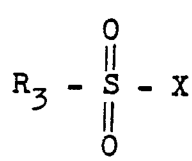

worin X ein Halogenatom ist und $R_3$ die in Anspruch 1 angegebene Bedeutung hat, reagiert und gegebenenfalls die erhaltene Verbindung durch Reaktion mit einer organischen Säure in das nichttoxische Salz überführt.

7. Ein Verfahren zur Herstellung einer Organosiliziumverbindung oder eines Salzes nach einem der Ansprüche 1, 2 oder 4, worin —$R_3$ —$CH_3$ ist, darin bestehend, daß man eine Verbindung (II) nach Anspruch

6 mit Ameisensäure und Essigsäureanhydrid behandelt, das Formylprodukt reduziert und gegebenenfalls die erhaltene Verbindung in das nichttoxische Salz überführt.

8. Ein Verfahren zur Herstellung einer Organosiliziumverbindung (I) nach den Ansprüchen 1, 2 oder 4, worin —A—$R_3$ Methyl und $R_4$ Wasserstoff ist, darin bestehend, daß man eine Verbindung, dargestellt durch die Formel:

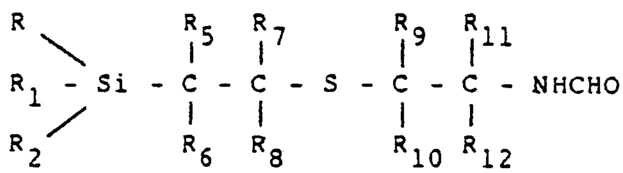

worin R, $R_1$, $R_2$, $R_5$, $R_6$, $R_7$, $R_8$, $R_9$, $R_{10}$, $R_{11}$ und $R_{12}$ die in den Ansprüchen 1 und 2 angegebenen Bedeutung haben, reduziert und gegebenenfalls die erhaltene Verbindung durch Reaktion mit einer organischen Säure in das nichttoxische Salz überführt.

9. Ein Verfahren zur Herstellung einer Organosiliziumverbindung oder eines Salzes nach den Ansprüchen 1, 2 oder 4, worin A Carbonyl ist, darin bestehend, daß man eine Verbindung, dargestellt durch die Formel

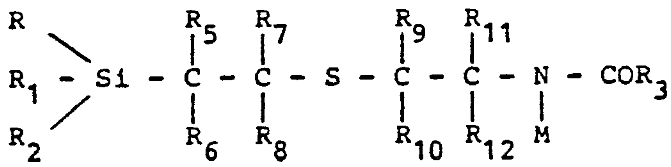
(VIII)

worin R, $R_1$, $R_2$, $R_3$, $R_5$, $R_6$, $R_7$, $R_8$, $R_9$, $R_{10}$, $R_{11}$ und $R_{12}$ die in den Ansprüchen 1 oder 2 angegebenen Bedeutungen haben, mit Natrium- oder Kaliumhydroxid reagiert, die erhaltene Verbindung, worin das N—H gleich N—Na oder N—K ist, mit einer Verbindung, dargestellt durch die Formel

$$R_4—X$$

worin X Halogen ist und $R_4$ eine Alkyl- oder Alkenylgruppe bedeutet, reagiert, und gegebenenfalls die erhaltene Verbindung durch Reaktion mit einer organischen Säure in das nichttoxische Salz überführt.

10. Ein Anti-Tumor-Agens bestehend aus einer wirksamen Komponente einer Organosiliziumverbindung oder eines Salzes nach einem der Ansprüche 1—4.

11. Ein Verfahren nach den Ansprüchen 5 oder 6, worin $R_4$ ein Wasserstoffatom ist.

12. Ein Verfahren nach Anspruch 5, worin $R_3$ eine Alkylgruppe oder eine substituierte Alkylgruppe ist.

13. Ein Verfahren nach Anspruch 6, worin $R_3$ eine Alkylgruppe, eine Phenylgruppe oder eine substituierte Phenylgruppe ist.

**Revendications**

1. Composé organosilicique de formule

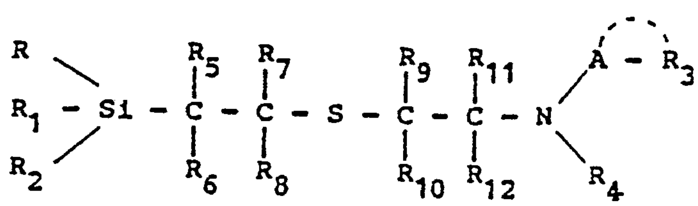
(I)

où

A représente un radical carbonyle ou sulfonyle;

R, $R_1$ et $R_2$ représentent chacun un radical alcoyle;

$R_3$ représente un radical alcoyle, trifluoroalcoyle, alcényle, phényle, p-chlorophényle, p-méthylphényle, naphtyle, pyridyle, furyle ou thiényle;

$R_4$ représente un atome d'hydrogène ou un radical alcoyle ou alcényle;

$R_5$, $R_6$, $R_7$, $R_8$, $R_9$, $R_{10}$, $R_{11}$ et $R_{12}$ représentent chacun un atome d'hydrogène ou un radical alcoyle; ou un sel non toxique de ce composé.

2. Composé organosilicique suivant la revendication 1, dans lequel R, $R_1$ et $R_2$ représentent chacun un radical méthyle et $R_5$, $R_6$, $R_7$, $R_8$, $R_9$, $R_{10}$, $R_{11}$ et $R_{12}$ représentent chacun un atome d'hydrogène.

3. Composé organosilicique suivant la revendication 1 ou 2, dans lequel $R_3$ représente un radical alcényle.

4. Maléate d'un composé organosilicique (I) suivant la revendication 1.

5. Procédé de préparation d'un composé organosilicique ou d'un sel de celui-ci tel que défini dans la revendication 1, où A est un radical carbonyle, qui comprend la réaction d'un composé de formule

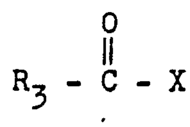
ou
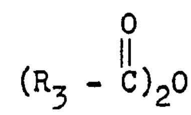

où X est un halogène et $R_3$ est tel que défini dans la revendication 1 ou 2, avec un composé de formule

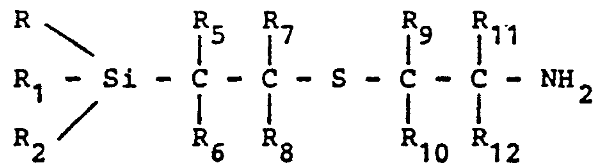
(II)

où R, $R_1$, $R_2$, $R_5$, $R_6$, $R_7$, $R_8$, $R_9$, $R_{10}$, $R_{11}$ et $R_{12}$ sont tels que définis dans la revendication 1 ou 2 et si nécessaire, la conversion du composé résultant en le sel non toxique par réaction avec un acide organique.

6. Procédé de préparation d'un composé organosilicique ou d'un sel tel que défini dans la revendication 1, où A est un radical sulfonyle, qui comprend la réaction d'un composé (II) tel que défini dans la revendication 5 avec un composé sulfonyle de formule

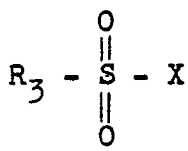

où X est un atome d'halogène et $R_3$ est tel que défini dans la revendication 1, et si nécessaire, la conversion du composé résultant en le sel non toxique par réaction avec un acide organique.

7. Procédé de préparation d'un composé organosilicique ou d'un sel tel que défini dans l'une quelconque des revendications 1, 2 et 4, dans lequel $R_3$ est un radical $—CH_3$, qui comprend la réaction d'un composé (II) tel que défini dans la revendication 6 avec l'acide formique et l'anhydride acétique, la réduction du produit formylé et, si nécessaire, la conversion du composé résultant en le sel non toxique.

8. Procédé de préparation d'un composé organosilicique (I) tel que défini dans la revendication 1, 2 ou 4, dans lequel $—A—R_3$ est un radical méthyle et $R_4$ est un atome d'hydrogène, qui comprend la réduction d'un composé de formule

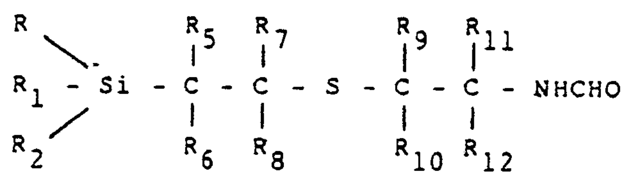

où R, $R_1$, $R_2$, $R_5$, $R_6$, $R_7$, $R_8$, $R_9$, $R_{10}$, $R_{11}$ et $R_{12}$ sont tels que définis dans la revendication 1 ou 2 et, si nécessaire, la conversion du composé résultant en le sel non toxique par réaction avec un acide organique.

9. Procédé de préparation d'un composé organosilicique ou d'un sel tel que défini dans la revendication 1, 2 ou 4, dans lequel A est un radical carbonyle, qui comprend la réaction d'un composé de formule

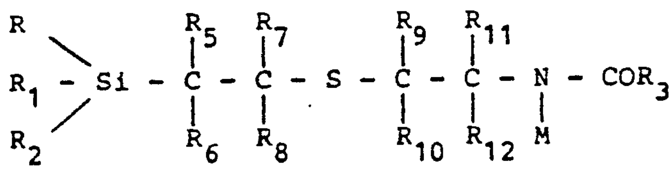
(VIII)

où R, $R_1$, $R_2$, $R_3$, $R_5$, $R_6$, $R_7$, $R_8$, $R_9$, $R_{10}$, $R_{11}$ et $R_{12}$ sont tels que définis dans la revendication 1 ou 2, avec l'hydroxyde de sodium ou de potassium, la réaction du composé résultant dans lequel N—H représente N—Na ou N—K avec un composé de formule

$R_4$—X

où X est un atome d'halogène et $R_4$ est un radical alcoyle ou alcényle et si nécessaire, la conversion du composé résultant en le sel non toxique par réaction avec un acide organique.

10. Agent antitumoral comprenant comme constituant efficace un composé organosilicique ou un sel tel que défini dans l'une quelconque des revendications 1 à 4.

11. Procédé suivant la revendication 5 ou 6, dans lequel $R_4$ est un atome d'hydrogène.

12. Procédé suivant la revendication 5, dans lequel $R_3$ est un radical alcoyle ou alcoyle substitué.

13. Procédé suivant la revendication 6, dans lequel $R_3$ est un radical alcoyle, phényle ou phényle substitué.